# DEMANDE DE BREVET EUROPEEN

(11) **EP 2 460 553 A1**
(43) Date de publication de la demande: **06.06.2012**
(21) Numéro de dépôt: 10193557.5
(22) Date de dépôt: 02.12.2010
(51) Int. Cl.: A61M 5/46, A61M 5/20, A61M 5/158, A61M 37/00

(54) **Dispositif pour l'insertion d'aiguilles**

(71) Demandeur: DEBIOTECH S.A., 1004 Lausanne (CH)
(72) Inventeur: Lemaire, Pierre, 1004, Lausanne (CH); Da Ros, Jérôme, 74200, Thonon les Bains (FR)
(74) Mandataire: Grosfillier, Philippe

(57) **Abrégé**

Dispositif pour l'insertion d'aiguilles comprenant un corps défini par une extrémité proximale et une extrémité distale (8), un support (3,7) monté mobile à l'intérieur du corps, au moins une aiguille (4) rendue solidaire du support (3,7) , des moyens d'entraînement (1,2) adaptés pour entraîner le support (3,7) vers ladite extrémité distale, ledit support (3,7) comportant une face distale sur laquelle l'aiguille (4) fait saillie, ladite extrémité distale (8) comportant une zone de contact destinée à contacter le tissu, le support (3,7) étant configuré de manière à atteindre une position, consécutivement à l'activation des moyens d'entraînement (1), dans laquelle sa face distale, par rapport à ladite extrémité proximale, est plus éloignée que ladite zone de contact ; le dispositif étant en outre configuré pour autoriser un recul passif du support (3,7) une fois ladite position atteinte.

## Description

### Domaine de l'invention

La présente invention concerne l'insertion d'aiguilles, en particulier de micro-aiguilles. Celle-ci peut être utilisée pour l'injection intradermique ou sous-cutanée de solutions.

### Etat de la technique

Des dispositifs pour l'insertion de micro-aiguilles sont divulgués dans les documents suivants : US 6 743 211, US 4 886 499, US 7 083 592 et US 20100030148.

### Description générale de l'invention

La présente invention constitue une amélioration par rapport aux méthodes et dispositifs de l'état de la technique. Elle se caractérise en particulier par le fait que le tissu peut être percuté par une ou plusieurs micro-aiguilles à des vitesses relativement élevées, typiquement de l'ordre de 7m/s, tout en laissant le tissu amortir le choc sur une certaine distance, ce qui a pour effet d'améliorer la perforation du tissu et de ramener cette dernière à un état stable d'équilibre, minimisant de la sorte les contraintes.

Dans le cadre de la présente invention, les micro-aiguilles sont libérées de tout appui physique lors de l'injection. La pression générée par celles-ci ne peut donc pas s'opposer à formation de papules induites par l'injection intradermique. Il est en outre possible de suivre la croissance du profil de la papule de façon à assurer la continuité de l'injection sans fuites.

Exprimé différemment, la présente invention se caractérise par une disparition partielle ou totale de la pression exercée par les aiguilles - ou de façon plus générale par le support des aiguilles - sur le tissu une fois que celles-ci y ont pénétré, ce qui a pour conséquence de faire revenir le tissu au plus proche de son état d'équilibre sans contrainte résiduelle.

La présente invention a donc pour objet un dispositif pour l'insertion d'aiguilles comprenant un corps défini par une extrémité proximale et une extrémité distale, un support monté mobile à l'intérieur du corps, au moins une aiguille rendue solidaire du support, des moyens d'entraînement adaptés pour entraîner le support vers ladite extrémité distale et un mécanisme d'activation des moyens d'entraînement, ledit support comportant une face distale sur laquelle l'aiguille fait saillie, ladite extrémité distale comportant une zone de contact destinée à contacter le tissu, le support étant configuré de manière à atteindre une position, consécutivement à l'activation des moyens d'entraînement, dans laquelle sa face distale, par rapport à ladite extrémité proximale, est plus éloignée que ladite zone de contact ; le dispositif étant en outre configuré pour autoriser un recul passif du support une fois ladite position atteinte. Dans une réalisation possible, le dispositif peut avoir un corps de forme allongée dans lequel, un piston est monté coulissant, l'aiguille faisant saillie sur ledit piston. Dans une réalisation possible, les moyens d'entraînement peuvent être un propulseur logeant un ressort, et entraînant le piston par contact.

Dans une réalisation possible, les aiguilles du dispositif sont des micro-aiguilles. Par micro-aiguilles on entend des aiguilles dont les dimensions sont adaptées pour cibler le territoire intra-dermique. Cette zone a une épaisseur variable selon le patient ainsi que selon l'emplacement sur le corps d'un même patient. Elle est de l'ordre de quelques centaines de microns. La micro-aiguille pourra cependant être un peu plus longue que l'épaisseur maximum de cette zone pour tenir compte de la pénétration des micro aiguilles dans le tissu qui pourrait n'être que partielle.

Dans le cadre de la présente invention la pression est minime du début à la fin de la formation de la papule. Une fois l'insertion effectuée, l'aiguille n'exerce comme force sur le tissu et la papule au maximum que son propre poids, celui du support et les éventuelles forces de frottement qui pourraient exister. L'appui physique pour fixer le tissu et maintenir le dispositif en place s'effectue loin du site d'injection et tout appui sur le dispositif n'est pas directement transmis à l'aiguille car le piston coulisse.

De préférence, le dispositif selon l'invention est dimensionné de manière à ce que lorsque qu'il est appliqué sur le tissu, la ou les aiguilles entrent en contact avec le tissu avant que le piston soit retenu par une butée. La longueur sur laquelle le piston est ainsi amorti par le tissu peut s'échelonner entre une fraction de la longueur de l'aiguille et 10mm.

Si le propulseur est retenu par une butée avant que la ou les aiguilles n'entrent en contact avec le tissu, la ou les aiguilles, connectées le cas échéant à une ligne d'injection utilisée pour délivrer une substance, peuvent se détacher du propulseur et continuer leur trajectoire indépendamment de celui-ci et ainsi pénétrer dans le tissu.

L'injection peut être déclenchée automatiquement ou manuellement une fois la ou les aiguilles insérées dans le tissu, mais préférentiellement elle commencera une fois le tissu revenu à son état d'équilibre. L'injection intradermique peut dans certains cas induire la formation d'une papule qui correspond à la déformation du tissu suite au stockage de la solution injectée dans le tissu, notamment lors d'une injection type bolus.

A relever que la création et le maintien d'une papule sont souhaitables pour assurer une injection optimale. C'est pourquoi la présente invention permet à une telle papule de se former et de rester en place le temps nécessaire à la bonne diffusion de la substance injectée.

Un mécanisme peut permettre de maintenir une force de maintien jusqu'au moment de l'injection où elle sera annulée.

A noter par ailleurs qu'avec la présente invention, l'appui du dispositif sur le tissu est indépendant de l'appui de l'aiguille ou des aiguilles sur le tissu.

A noter par ailleurs que le système d'appui du dispositif a aussi comme rôle de fixer la distance qui sépare la ou les aiguilles de la surface du tissu avant l'activation dudit dispositif.

L'invention concerne également une méthode d'insertion et d'injection grâce à une ou des aiguilles, notamment de micro-aiguilles et notamment dans le derme, où l'aiguille dotée d'un mouvement de translation peut décélérer dans le tissu du fait de l'élasticité de celui-ci sur une certaine longueur tout en permettant *in fine* un retour partiel ou total en arrière à l'état d'équilibre (ou proche de celui-ci) du simple effet de l'élasticité du tissu. L'aiguille peut donc librement se mouvoir sur une certaine distance, qui comprend la surface du tissu. L'aiguille peut se déplacer dans le tissu et pousser cette dernière au-delà de sa hauteur initiale à l'équilibre. De manière analogue, une fois l'aiguille partiellement insérée dans le tissu, une limitation dure, partielle ou progressive peut l'empêcher de repartir en arrière (contre-force), au-delà ou en deçà de la hauteur naturelle (initiale) d'équilibre. L'aiguille va se positionner à l'équilibre de la force élastique du tissu et cette potentielle contre-force. Préférentiellement, l'équilibre après insertion doit être le plus proche possible de l'équilibre naturel du tissu, voire avec une légère contre-force pour empêcher le retrait de l'aiguille du tissu. Préférentiellement, la contre-force doit empêcher que le rétablissement à son équilibre du tissu ne fasse ressortir l'aiguille par inertie, mais cette contre-force ne doit pas empêcher l'injection. En particulier cette contre-force ne doit pas créer de pression à l'intérieur du tissu qui empêcherait ou limiterait la bonne diffusion de la substance devant être infusée.

Lors de l'injection l'aiguille reste solidaire de la papule en se déplaçant avec elle lors de sa croissance, tout en opposant un minimum de résistance. Une légère contre-force, en plus du poids en cas de position verticale, peut être présente pour assurer un maintien de l'ensemble.

De préférence, le propulseur est mis en mouvement par le relâchement d'une énergie potentielle, dans notre cas, un ressort. Cette énergie peut être de forme variée, ressort plastique, ressort à lamelle, cartouche de gaz, air comprimé, force électro-magnétique, génération de gaz par réaction chimique entre au moins deux composés. Le piston peut être directement mis en mouvement sans l'aide d'un propulseur, seulement à l'aide de la source d'énergie. La source d'énergie peut être l'utilisateur lui-même. En appuyant avec une force suffisante, il générera par un mécanisme interne au dispositif la vitesse nécessaire à la pénétration de la ou des aiguilles dans le tissu.

Le dispositif comprend également un mécanisme de sécurité, permettant de verrouiller le moyen d'activation afin d'éviter un déclenchement involontaire, dans le cas présenté, il s'agit d'un second bouton de sécurité à activer avant d'appuyer sur la détente qui a pour but de provoquer l'insertion. Le mécanisme de sécurité peut se présenter sous différentes formes, l'appui de la bague ou du dispositif sur le tissu peut également déverrouiller la détente de déclenchement.

De préférence, le dispositif comprend aussi un récipient utilisé pour stocker substance à administrer. Ce récipient peut être positionné sur le corps du dispositif ou être directement intégré au dispositif. L'activation de ce récipient, qui provoquera l'injection de la substance dans le tissu, peut être soit manuelle, soit automatique. Dans ce deuxième cas, l'activation du dispositif entraînera, une fois les aiguilles en place, l'activation du récipient. Ce récipient peut prendre diverses formes telles que - et ceci de façon non exhaustive - un réservoir semi rigide, une poche souple, une seringue, une carpule.

Le dispositif peut comprendre un mécanisme qui rend difficilement accessible l'aiguille de manière à prévenir les blessures, avant ou après l'insertion ainsi qu'après l'injection quand le dispositif est utilisé dans ce but. Cette protection peut s'obtenir notamment en rétractant l'aiguille dans le corps du dispositif, ou dans le support lui-même, ou en coulissant un cache qui empêche l'accès à l'aiguille.

L'utilisation du dispositif n'est pas restreinte à une position verticale.

De préférence, le dispositif est orienté pour permettre une pénétration des aiguilles perpendiculairement au tissu. Dans certaines réalisations, cet angle peut cependant être modifié.

### Description détaillée de l'invention

L'invention sera décrite de manière détaillée ci-dessous au moyen d'exemples non-limitatifs illustrés par des figures.

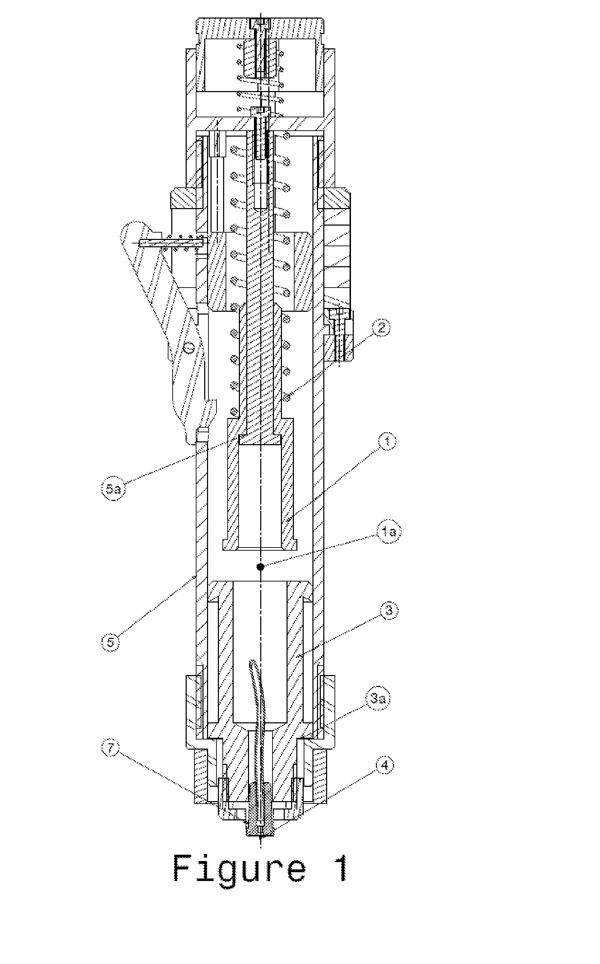

Figure 1 : Représentation d'un mode de réalisation d'un dispositif selon l'invention. Une aiguille (4) est rendue solidaire d'un piston (3) par l'intermédiaire d'un connecteur (7). Le propulseur (1) est relâché en arrière du piston (3), l'espace libre (1 a) de coulissage est visible entre les deux.

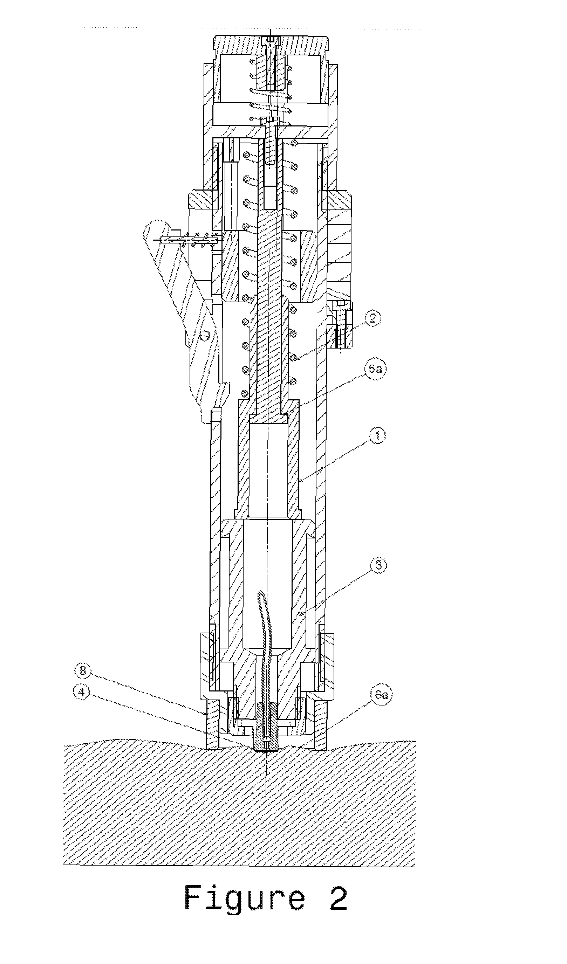

Figure 2 : Dans cette configuration préférentielle, le dispositif est posé sur le tissu (6a) par le contact avec une bague extérieure (8). Le dispositif a été armé et déclenché. Le propulseur (1) a poussé le piston (3) et vient d'entrer en butée (5a) (cf. tige dans le propulseur). Le piston (3) va continuer sa course du fait de l'inertie. Pour cette configuration, l'instant où le piston (3) va se détacher du propulseur (1) correspond en moyenne à l'instant où l'aiguille (4) commence à pénétrer le tissu.

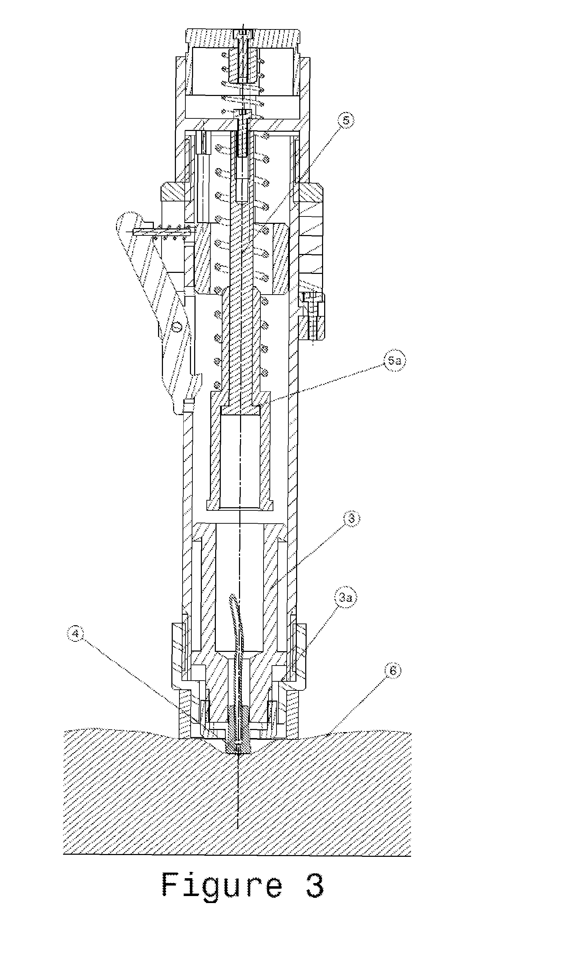

Figure 3 : Même dispositif que celui de la figure 2. Le piston (3) a continué sa course avec l'inertie, l'aiguille (4) pénètre et compresse le tissu (6) de plus en plus au fur et à mesure que la force élastique de rappel du tissu (6) augmente avec la déformation, le piston (3) décélère jusqu'à ce que la force d'inertie devienne inférieure à la force élastique, l'insertion de l'aiguille (4) est alors maximale. Le mouvement s'inverse alors avec le tissu revenant à son équilibre initial « naturel » (cf. 6a figure2)

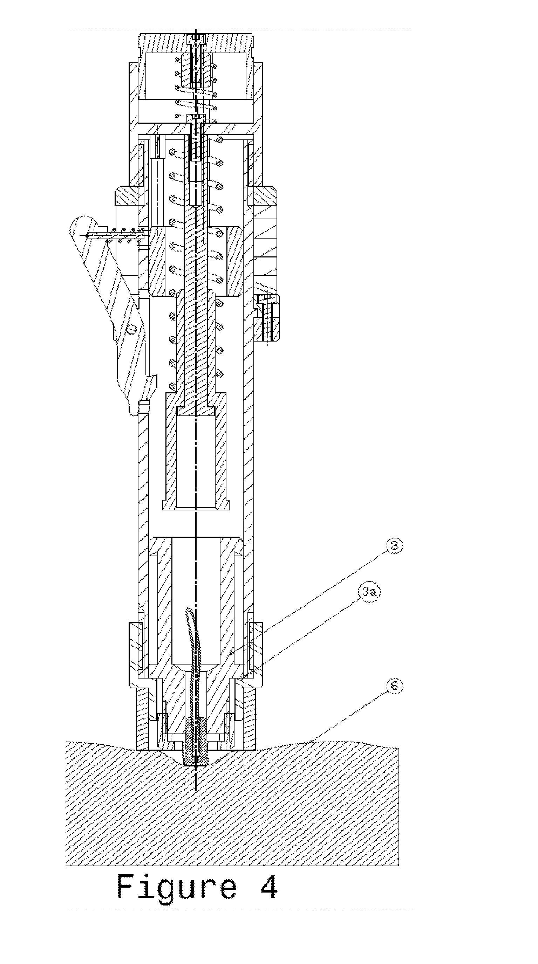

Figure 4 : Selon un autre mode de réalisation le dimensionnement est tel que le piston (3) rencontre une butée franche (3a) sur le corps du dispositif avant que la force d'inertie s'équilibre avec la force élastique du tissu (6). On peut également imaginer une butée partielle ou progressive type force de rappel.

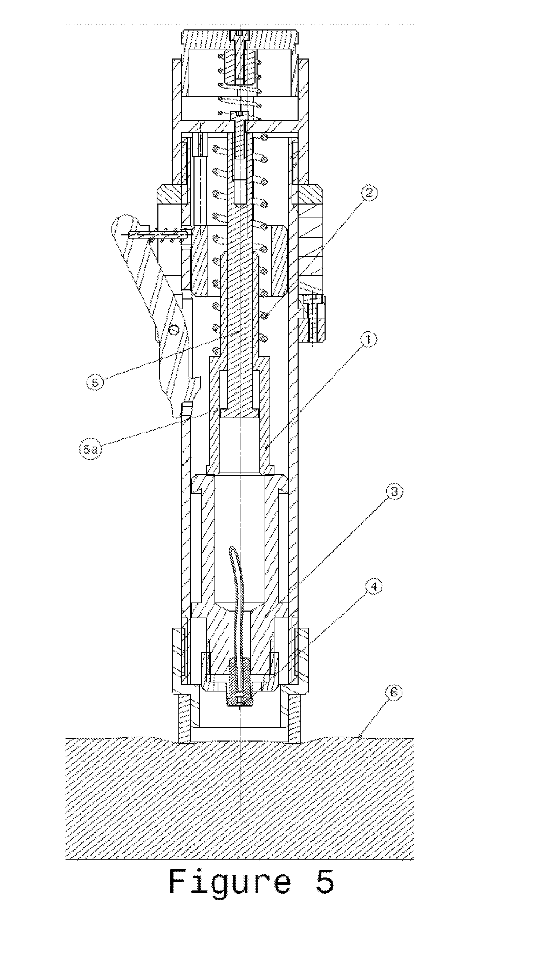

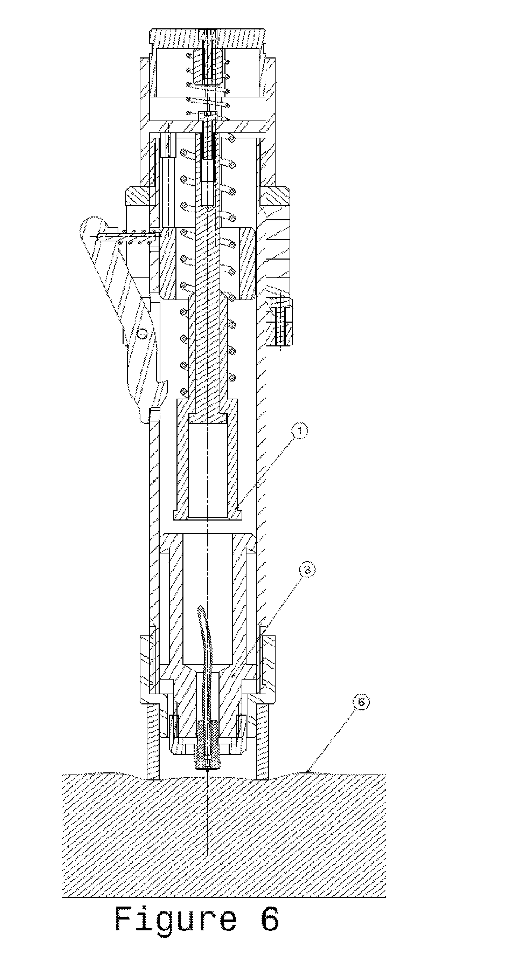

Figures 5 et 6 : Dans une autre variante de l'invention, le dimensionnement est tel que le piston (3) se détache du propulseur alors que l'aiguille n'a pas encore contacté le tissu.

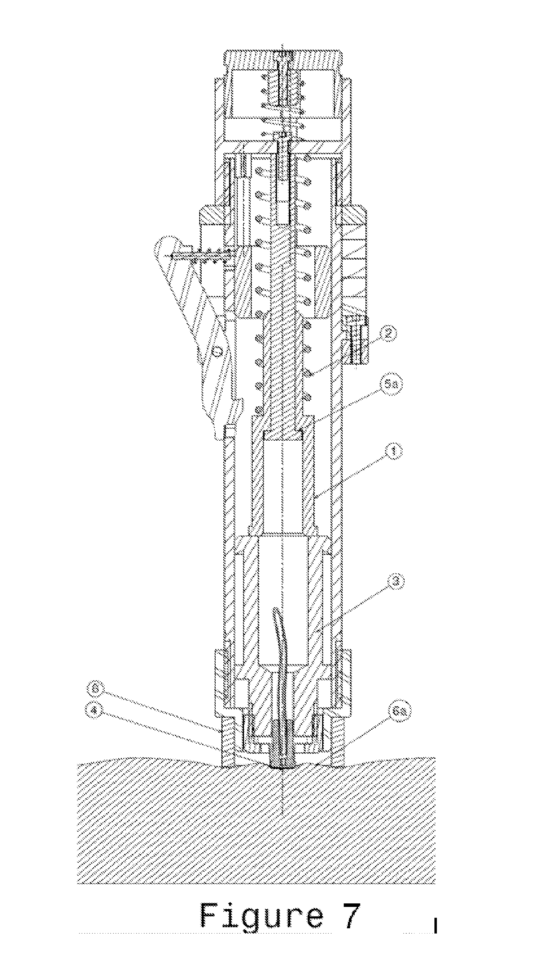

Figure 7: La force élastique renvoie à sa position initiale le piston (3), les aiguilles (4) restant bien insérées dans le tissu (6a), le propulseur (1) offre alors une force de rappel atténuant le régime oscillatoire et empêchant par inertie le piston (3) de rebondir et faire sortir l'aiguille (4) du tissu (6). L'équilibre est atteint avec les déformations imposées au tissu (6) relâchées mais avec l'aiguille bien insérée. Une légère force résiduelle de la part du tissu et une légère contre-force de la part du propulseur (1) peuvent être adéquat pour maintenir en place l'aiguille (4).

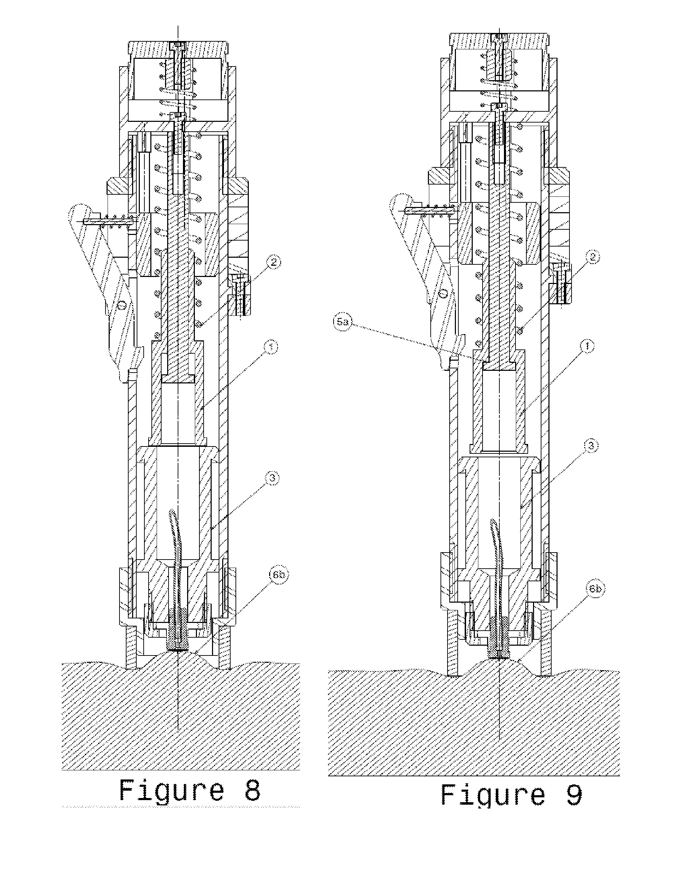

Figure 8 : L'injection peut commencer, préférentiellement à l'équilibre, la papule (6b) en formation pousse le piston (3) qui peut coulisser sans offrir de forte résistance et en permettant à l'aiguille (4) de rester bien insérée dans la papule (6b) en guidant le mouvement. Ici le ressort (2) du propulseur est en fin de compression et n'offre qu'une faible contre-force à la formation de la papule (6b), en plus du poids faible de l'ensemble piston (3) connecteur(7) aiguille (4) tube.
Figure 9 : Selon cette variante, la contre-force du propulseur (1) ne s'applique pas, la hauteur prise par le piston (3) du fait de la formation de la papule (6b) étant inférieure à la distance libre entre le propulseur (1) en butée (5a) et le piston(3).

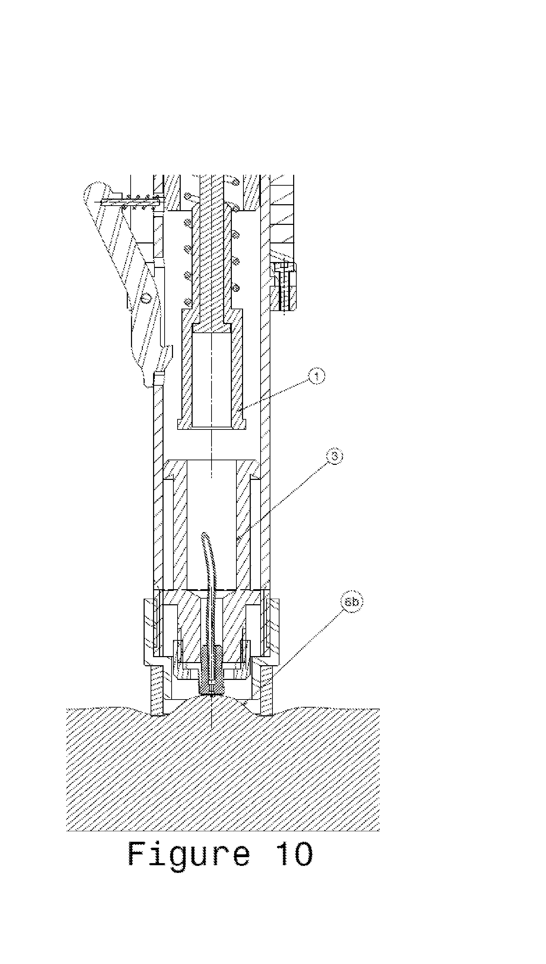

Figure 10 : Selon cette autre variante, à l'équilibre le propulseur (1) est éloigné (p.ex. recomprimé, ressort relâché par le haut, étage propulseur relevé) pour laisser l'espace libre au piston (3) pour remonter avec la formation de la papule (6b). Il s'agit de se retrouver dans la configuration du lanceur pour l'injection, mais tout en assurant la contre-force à la force élastique lors de l'insertion.

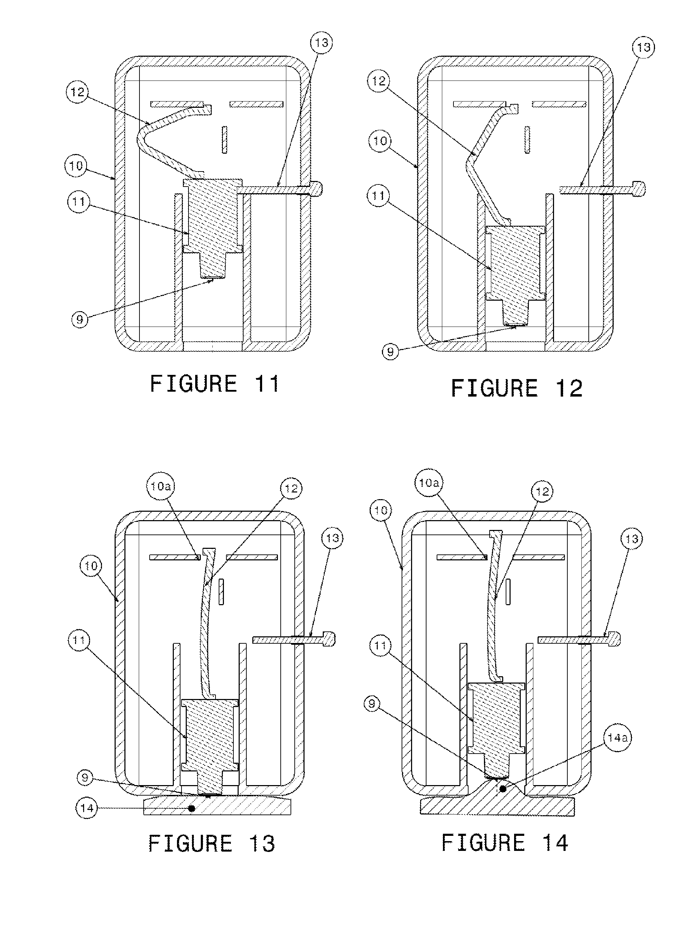

Figure 11 : Représentation d'un mode de réalisation d'un dispositif selon l'invention. Une aiguille (9) est rendue solidaire d'un piston (11). Une lamelle ressort (12) est mise sous contrainte entre le piston (11) et le boîtier (10a).
Figure 12-13: Dans cette configuration, le dispositif est posé sur le tissu. Le dispositif a été déclenché. Le ressort (12) se détend et pousse le piston (11) qui vient d'entrer en butée avec le tissu (14), Le piston (11) va continuer sa course du fait de l'inertie. Pour cette configuration, l'instant où la lamelle ressort va se délier du boîtier (10a), correspond en moyenne à l'instant où l'aiguille (9) commence à pénétrer le tissu (14).
Figure 14 : L'injection peut commencer, préférentiellement à l'équilibre, la papule (14a) en formation pousse le piston (11) qui peut coulisser sans offrir de forte résistance et en permettant à l'aiguille (9) de rester bien insérée dans la papule (14a) en guidant le mouvement. Ici le ressort (12) est décomprimé et n'offre aucune contre-force à la formation de la papule (14a).

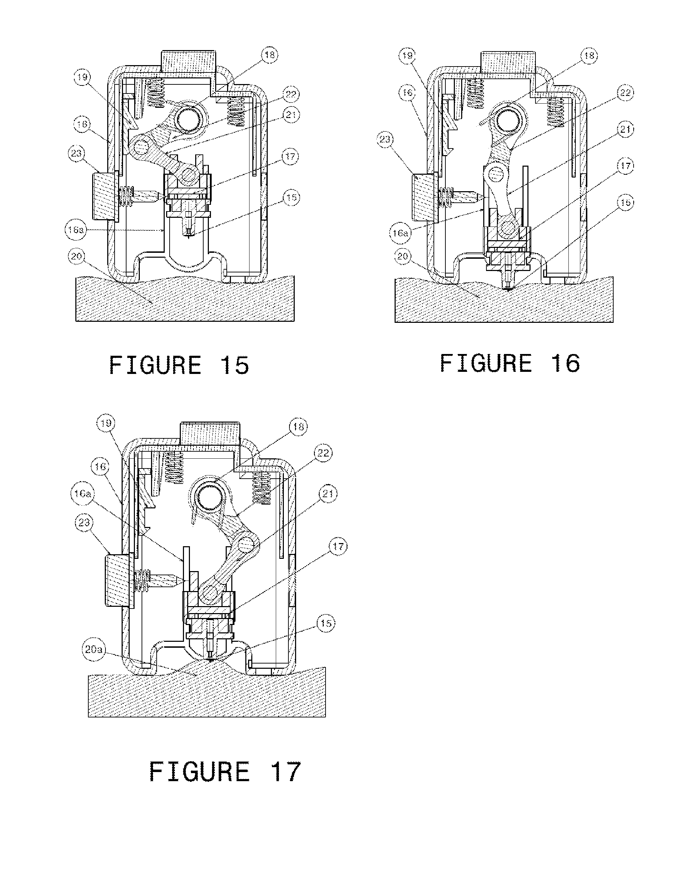

Figure 15 : Représentation d'un mode de réalisation d'un dispositif selon l'invention. Une aiguille (15) est rendue solidaire d'un piston (17). Un système mécanique « bielle-manivelle » (21,22) permet de transformer le mouvement de rotation activé par le ressort (18) en translation au piston (17). Le piston (17) peut coulisser dans les guides du boîtier (16a).
Figure 16: Dans cette configuration, le dispositif est posé sur le tissu. Le dispositif a été déclenché. Le ressort (18) se détend et active le système « bielle-manivelle » (21,22) qui pousse le piston (17) qui vient d'entrer en butée avec le tissu (16), Le piston (11) va continuer sa course du fait de l'inertie. Pour cette configuration, l'instant où le ressort est détendu correspond en moyenne à l'instant où l'aiguille (15) commence à pénétrer le tissu (20). Pour une autre configuration le ressort permet de garder une certaine force du piston (17) sur le tissu (20), le fait d'appuyer sur le bouton permet de libérer l'effort exercé par le piston (17) sur le tissu (20).
Figure 17 : L'injection peut commencer, préférentiellement à l'équilibre, la papule (20a) en formation pousse le piston (17) qui peut coulisser sans offrir de forte résistance et en permettant à l'aiguille (15) de rester bien insérée dans la papule (20a) en guidant le mouvement. Ici le ressort (18) est décomprimé et n'offre aucune contre-force à la formation de la papule (20a), le système « bielle-manivelle » (21,22) autorise un retour du piston (17) lors de la formation de la papule (20a).

## Revendications

1. Dispositif pour l'insertion d'aiguilles comprenant un corps défini par une extrémité proximale et une extrémité distale (8), un support (3,7) monté mobile à l'intérieur du corps, au moins une aiguille (4) rendue solidaire du support (3,7), des moyens d'entraînement (1,2) adaptés pour entraîner le support (3,7) vers ladite extrémité distale, ledit support (3,7) comportant une face distale sur laquelle l'aiguille (4) fait saillie, ladite extrémité distale (8) comportant une zone de contact destinée à contacter le tissu, le support (3,7) étant configuré de manière à atteindre une position, consécutivement à l'activation des moyens d'entraînement (1), dans laquelle sa face distale, par rapport à ladite extrémité proximale, est plus éloignée que ladite zone de contact ; le dispositif étant en outre configuré pour autoriser un recul passif du support (3,7) une fois ladite position atteinte.

2. Dispositif selon la revendication 1 dans lequel le support (3,7) est formé d'un piston (3) et d'un connecteur (7), ladite face distale étant disposée sur le connecteur (7).

3. Dispositif selon la revendication 1 ou 2 dans lequel le corps comporte un premier élément de butée (3a) disposé de manière à retenir le support (3,7) lorsqu'il est entraîné vers ladite extrémité distale.

4. Dispositif selon l'une des revendications précédentes dans lequel le corps comporte un deuxième élément de butée (5a) disposé de manière à retenir les moyens d'entraînement (1,2) lorsqu'ils sont entraînés vers ladite extrémité distale.

5. Dispositif selon l'une des revendications 2 à 4 dans lequel les moyens d'entraînement (1,2) sont en contact mécanique avec le piston (3) lorsque celui-ci se déplace vers ladite extrémité distale et qu'un espace libre (1a) est formé pendant la phase de recul du piston (3).

6. Dispositif selon l'une des revendications précédentes dans lequel les moyens d'entraînement (1,2) comprennent un ressort (2).

7. Dispositif selon l'une des revendications 2 à 6 dans lequel plusieurs aiguilles (4) sont rendues solidaires du piston (3) par l'intermédiaire du connecteur (7).

8. Dispositif selon l'une des revendications 2 à 7 comprenant des moyens de retenue du piston (3) qui sont disposés de manière à autoriser le recul du piston (3) sur une distance prédéfinie.

9. Dispositif selon l'une des revendications précédentes dans lequel un conteneur pouvant contenir une substance à délivrer au travers de l'aiguille fait partie intégrante du dispositif, est placé sur le corps du dispositif ou est lié directement à un élément mobile.

10. Dispositif selon l'une des revendications précédentes comprenant un mécanisme d'activation des moyens d'entraînement (1,2).

11. Dispositif selon l'une des revendications précédentes comprenant un mécanisme de sécurité qui verrouille les moyens d'entraînement (1,2) ou le mécanisme d'activation des moyens d'entraînement (1,2).

12. Dispositif selon l'une des revendications précédentes comprenant un mécanisme qui rend difficilement accessible l'aiguille de manière à prévenir les blessures, avant ou après l'utilisation.

13. Dispositif selon la revendication 12 où l'aiguille est rétractée dans le corps du dispositif.

14. Dispositif selon la revendication 12 où un cache rétractable empêche l'accès à l'aiguille.

15. Utilisation d'un ensemble pour l'insertion d'aiguilles et l'injection de solutions comprenant les étapes suivantes :
a) insertion d'au moins une aiguille dans ou à travers le tissu,
b) première rétraction passive de l'aiguille résultant d'un retour à l'état d'équilibre du tissu,
c) injection d'au moins une solution à travers l'aiguille.

16. Utilisation selon la revendication 15 dans laquelle lesdites étapes sont réalisées successivement.

17. Utilisation selon la revendication 15 dans laquelle les étapes b) et c) sont réalisées simultanément.

18. Utilisation selon l'une des revendications 15 à 17 comprenant en outre l'étape suivante :
d) deuxième rétraction passive de l'aiguille résultant de la formation d'une papule consécutivement à l'étape c).
